# EUROPÄISCHE PATENTSCHRIFT

(11) **EP 2 736 561 B1**
(45) Veröffentlichungstag und Bekanntmachung des Hinweises auf die Patenterteilung: **06.01.2016**
(21) Anmeldenummer: 12743676.4
(22) Anmeldetag: 27.07.2012
(51) Int. Cl.: A61M 1/36

(54) **VERFAHREN SOWIE VORRICHTUNGEN ZUM ABLÖSEN VON GASANSAMMLUNGEN VON EINEM GERINNSELFÄNGER EINES EXTRAKORPORALEN BLUTKREISLAUFS**
METHOD AND DEVICES FOR DETACHING GAS ACCUMULATIONS FROM A BLOOD CLOT CATCHER OF AN EXTRACORPOREAL BLOOD CIRCULATION
PROCÉDÉ ET DISPOSITIFS POUR DÉTACHER DES AMAS GAZEUX D'UN PIÈGE À CAILLOTS D'UNE CIRCULATION SANGUINE EXTRA-CORPORELLE

(30) Priorität: 29.07.2011 DE 102011110472; 29.07.2011 US 201161512933 P
(43) Veröffentlichungstag der Anmeldung: 04.06.2014
(73) Patentinhaber: Fresenius Medical Care Deutschland GmbH, 61352 Bad Homburg (DE)
(72) Erfinder: GRONAU, Soeren, 64569 Nauheim (DE); NIKOLIC, Dejan, 60599 Frankfurt (DE); NOACK, Joachim, 97616 Bad Neustadt (DE)
(74) Vertreter: Bobbert, Cornelius
(86) Internationale Anmeldenummer: PCT/EP2012/003192
(87) Internationale Veröffentlichungsnummer: WO 2013/017238

(56) Entgegenhaltungen:
- EP-A2- 0 876 822
- WO-A1-01/32256
- DE-A1-102009 024 468
- US-A- 4 026 669
- US-A1- 2010 270 230

## Beschreibung

Die vorliegende Erfindung betrifft ein Verfahren gemäß Anspruch 1. Sie betrifft zudem eine Steuerungseinrichtung gemäß Anspruch 12 und eine medizinische Behandlungsvorrichtung gemäß Anspruch 13.

Extrakorporale Blutschlauchsysteme, wie sie z. B. in der Dialyse oder beim Einsatz von Herzlungenmaschinen zum Einsatz kommen, verfügen häufig über Einrichtungen zum Zurückhalten von Blutgerinnseln. Diese sogenannten Gerinnselfänger befinden sich üblicherweise auf der Ausgangsseite des Blutschlauchsystems und sind regelmäßig als feinmaschige Gewebe oder Gitter ausgestaltet. An ihren Maschen können sich Gasblasen verfangen, was unerwünscht ist. Zum Verhindern oder Reduzieren des Verfangens von Gasblasen am Gerinnselfänger und/oder zum Ablösen von Gasansammlungen oder Gasanhaftungen vom Gerinnselfänger sind Verfahren und konstruktive Maßnahmen wie z.B. aus DE 10 2009 024468 bekannt.

Eine Aufgabe der vorliegenden Erfindung ist es, ein weiteres Verfahren zum Ablösen von Gasblasen oder Gasansammlungen von einem Gerinnselfänger oder einer anderen Komponente eines extrakorporalen Blutkreislaufs vorzuschlagen. Zudem sollen geeignete Ein- bzw. Vorrichtungen, ein geeignetes digitales Speichermedium, ein geeignetes Computerprogramm-Produkt und ein geeignetes Computerprogramm angegeben werden.

Die erfindungsgemäße Aufgabe wird durch ein Verfahren mit den Merkmalen des Anspruchs 1 gelöst. Sie wird ferner gelöst mittels der Steuerungseinrichtung mit den Merkmalen des Anspruchs 15, der Behandlungsvorrichtung mit den Merkmalen des Anspruchs 16, des digitalen Speichermediums mit den Merkmalen des Anspruchs 18, des Computerprogramm-Produkts mit den Merkmalen des Anspruchs 19 sowie des Computerprogramms mit den Merkmalen des Anspruchs 20.

Erfindungsgemäß wird somit ein Verfahren zum Ablösen von Gasansammlungen von (oder aus) einem Gerinnselfänger oder einer anderen Komponente eines extrakorporalen Blutkreislaufs, welcher während einer extrakorporalen Blutbehandlung in einer ersten Strömungsrichtung durchströmt oder angeströmt wird, vorgeschlagen. Das erfindungsgemäße Verfahren umfasst das Erzeugen eines Stroms eines Fluids durch die Komponente, beispielsweise den Gerinnselfänger, hindurch - oder in ihn hinein - in einer zweiten Strömungsrichtung.

Die erfindungsgemäße Steuerungseinrichtung ist geeignet und vorgesehen und/oder ausgelegt und/oder konfiguriert zur Durchführung des erfindungsgemäßen Verfahrens.

Die erfindungsgemäße medizinische oder medizintechnische Behandlungsvorrichtung (im Folgenden auch kurz: Behandlungsvorrichtung) weist wenigstens eine Steuerungseinrichtung auf und/oder ist hiermit in Signalübertragung verbunden oder steht mit dieser in einer Signalübertragungsbeziehung.

Ein erfindungsgemäßes digitales , insbesondere nichtflüchtiges, Speichermedium, insbesondere in Form eines maschinenlesbaren Trägers, insbesondere in Form einer Diskette, CD oder DVD oder EPROM, mit elektronisch oder optisch auslesbaren Steuersignalen kann derart mit einem programmierbaren Computersystem zusammenwirken, dass die maschinellen Schritte des erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode zur Veranlassung der maschinellen Schritte des erfindungsgemäßen Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein erfindungsgemäßes Computerprogramm weist einen Programmcode auf zur Veranlassung der maschinellen Schritte des erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In manchen erfindungsgemäßen Ausführungsformen ist eine Gasansammlung Luft oder ein anderes Gasgemisch.

In einigen erfindungsgemäßen Ausführungsformen ist das Fluid eine Flüssigkeit, vorzugsweise Dialysierflüssigkeit oder Substituat. In manchen erfindungsgemäßen Ausführungsformen ist das Fluid eine Hydraulikflüssigkeit, beispielsweise aus der Compliance der Hydraulik (Dialyatseite) der Behandlungsvorrichtung.

In bestimmten erfindungsgemäßen Ausführungsformen erfolgt das Erzeugen eines Stroms eines Fluids durch die Durchtrittsöffnungen des Gerinnselfängers hindurch.

Die zweite Strömungsrichtung ist in manchen erfindungsgemäßen Ausführungsformen nicht die erste Strömungsrichtung. In gewissen erfindungsgemäßen Ausführungsformen ist die zweite Strömungsrichtung der ersten Strömungsrichtung entgegensetzt. In einigen erfindungsgemäßen Ausführungsformen strömt ein sich in der zweiten Strömungsrichtung bewegendes Fluid in die venöse Blutkammer hinein oder auf diese zu.

In manchen erfindungsgemäßen Ausführungsformen umfasst das Verfahren das Aufbauen eines Drucks mittels des Fluids zu einem Zeitpunkt, welcher vor dem Zeitpunkt des Erzeugens des Stroms oder des Beginns des Stroms in die zweite Strömungsrichtung liegt. In diesen Ausführungsformen wird zunächst ein Druck aufgebaut, dessen Ausgleich oder zumindest teilweiser Abbau erst nach Erreichen eines vorbestimmten oder gewünschten Druckniveaus zugelassen wird, und zwar mittels oder unter Erzeugen eines Fluidstroms in der zweiten Strömungsrichtung. Das Aufbauen eines Drucks mittels des Fluids erfolgt somit zeitlich vor Erzeugen des Stroms in die zweite Strömungsrichtung.

Der auf diese Weise aufgebaute Druck wird in einigen erfindungsgemäßen Ausführungsformen vor einem Ventil aufgebaut.

Der mittels des Fluids aufgebaute Druck ist in einigen erfindungsgemäßen Ausführungsformen ein positiver Druck.

In manchen erfindungsgemäßen Ausführungsformen ist dieser Druck ein Überdruck. In einigen erfindungsgemäßen Ausführungsformen liegt dieser Druck über einem üblichen oder durchschnittlichen Betriebsdruck, welcher bei Durchströmen des Gerinnselfängers in der ersten Strömungsrichtung oder während der Blutbehandlung herrscht.

In anderen erfindungsgemäßen Ausführungsformen ist der Druck ein Unterdruck, beispielsweise über die Membran des Blutfilters hinweg.

Der Unterdruck kann ein Druckabfall ausgehend vom Blutkompartment des Blutfilters in Richtung zum Dialysatkompartment des Blutfilters sein.

Der Unterdruck kann durch eine Saugwirkung der Hydraulik der Behandlungsvorrichtung entstehen oder entstanden sein.

In gewissen erfindungsgemäßen Ausführungsformen liegt der Druck über einem mittels der Fördereinrichtung während der Blutbehandlung erzeugten Druck oder Durchschnittsdruck.

Das Aufbauen eines Drucks mittels des Fluids entspricht in einigen erfindungsgemäßen Ausführungsformen einem plötzlichen Druckanstieg im oder am Gerinnselfänger. Ein plötzlicher Druckanstieg ist in bestimmten Ausführungsformen ausschließlich auf den Einsatz der Fördereinrichtung zurückzuführen, in anderen Ausführungsformen hingegen nicht.

In einigen erfindungsgemäßen Ausführungsformen umfasst das Verfahren das Erzeugen des Stroms in der zweiten Richtung und/oder das Aufbauen des Drucks unter Einsatz einer Blutpumpe. In diesen oder in anderen erfindungsgemäßen Ausführungsformen umfasst das Verfahren das Erzeugen des Stroms in der zweiten Richtung und/oder das Aufbauen des Drucks unter Einsatz einer Hydraulikeinrichtung einer medizinischen Behandlungsvorrichtung. "Unter Einsatz" kann hier als "mittels" verstanden werden.

In einigen erfindungsgemäßen Ausführungsformen umfasst das Verfahren das Schließen wenigstens eines Ventils zur Unterstützung des Aufbaus des Drucks.

In bestimmten erfindungsgemäßen Ausführungsformen ist das Schließen ein aktiver Vorgang, und/oder das Schließen erfolgt zum Zwecke der Unterstützung des Aufbaus des Drucks.

Das wenigstens eine zu schließende Ventil ist in manchen erfindungsgemäßen Ausführungsformen eine venöse Patientenschlauchklemme.

In bestimmten erfindungsgemäßen Ausführungsformen umfasst das Verfahren das Kurzschließen eines arteriellen Abschnitts des extrakorporalen Blutkreislaufs mit einem venösen Abschnitt des extrakorporalen Blutkreislaufs. In anderen erfindungsgemäßen Ausführungsformen umfasst das Verfahren ein solches Kurzschließen oder Verbinden nicht.

In manchen erfindungsgemäßen Ausführungsformen umfasst das Verfahren das Überwachen der Höhe des aufgebauten Drucks und/oder des Druckgradienten mittels einer geeigneten Einrichtung.

Das Überwachen der Höhe des aufgebauten Drucks und/oder des Druckgradienten geht in gewissen erfindungsgemäßen Ausführungsformen mit einem Begrenzen der Höhe einher oder umfasst ein solches.

In einigen erfindungsgemäßen Ausführungsformen erfolgt das Überwachen der Höhe des aufgebauten Drucks und/oder des Druckgradienten durch Öffnen des Ventils oder der Klemme bei Erreichen einer vorbestimmten maximalen Höhe des Drucks.

Die Einrichtung zum Überwachen der Höhe des aufgebauten Drucks und/oder des Druckgradienten weist in bestimmten erfindungsgemäßen Ausführungsformen einen Drucksensor auf oder besteht aus einem solchen.

In manchen erfindungsgemäßen Ausführungsformen umfasst das Verfahren das Öffnen des Ventils der Zugabestelle für Substituatflüssigkeit zur Prädilution und/oder des Ventils der Zugabestelle für Substituatflüssigkeit zur Postdilution. Ihr Öffnen kann vorteilhaft dazu beitragen, den Strömungswiderstand des Blutfilters oder Dialysators vorteilhaft zu umgehen.

In bestimmten erfindungsgemäßen Ausführungsformen umfasst das Verfahren das Öffnen des Single-Needle-Ventils, in manchen erfindungsgemäßen Ausführungsformen das Öffnen des Entlüftungsventils des extrakorporalen Blutkreislaufs. Ihr Öffnen kann vorteilhaft beitragen zu vermeiden, dass sich auf der Saugseite der Fördereinrichtung ein großer oder gar zu großer Unterdruck aufbaut. In anderen erfindungsgemäßen Ausführungsformen bleiben Single-Needle-Ventil und/oder Entlüftungsventil des extrakorporalen Blutkreislaufs geschlossen. Dies kann vorteilhaft zum Erzielen hoher Spitzendrücke und/oder eines hohen Druckgradienten über dem Gerinnselfänger beitragen.

In gewissen erfindungsgemäßen Ausführungsformen umfasst das Verfahren ein zwei- oder mehrfaches Durchführen aller zuvor genannten Schritte.

Das Verfahren umfasst in manchen erfindungsgemäßen Ausführungsformen das Abbrechen des Verfahrens nach einer vorbestimmten Anzahl von durchgeführten Zyklen und/oder nachdem ein vorbestimmtes Volumen an Fluid mittels der Fördereinrichtung in der zweiten Strömungsrichtung gefördert wurde.

In bestimmten erfindungsgemäßen Ausführungsformen erfolgt das Verfahren vor Beginn einer Blutbehandlung, beispielsweise im Zusammenhang mit dem Spülen oder Primen des extrakorporalen Blutkreislaufs.

"Vor Beginn einer Blutbehandlung" bedeutet in einigen erfindungsgemäßen Ausführungsformen, dass das erfindungsgemäße Verfahren vor Beginn der Behandlung des Patienten beendet ist.

In gewissen erfindungsgemäßen Ausführungsformen weist die Steuerungseinrichtung Stelleinrichtungen, Messeinrichtungen, Steuereinrichtungen oder Regeleinrichtungen, Auswerteinrichtungen, Vergleichseinrichtungen, und/oder Speichereinrichtungen für Vergleichsdaten auf.

Die erfindungsgemäße Behandlungsvorrichtung ist in manchen Ausführungsformen als Blutbehandlungsvorrichtung, insbesondere als Vorrichtung zur Apherese oder Dialyse, wiederum insbesondere zur Hämodialyse, Hämofiltration, Hämodiafiltration, oder zur Akutdialyse ausgestaltet.

In einigen erfindungsgemäßen Ausführungsformen der Behandlungsvorrichtung ist die Fördereinrichtung als Blutpumpe ausgestaltet. In bestimmten erfindungsgemäßen Ausführungsformen ist sie als Rollenpumpe ausgestaltet.

Die Erfindung bezieht sich auf eine Schlauchkonfiguration und ein Verfahren, welches die Entlüftung von Gerinnselfängern vorteilhaft erleichtert: Das Verfahren basiert darauf, dass die Strömungsrichtung z. B. beim Füllen/Spülen des Disposables im Gerinnselfänger vorübergehend umgekehrt wird.

Ein Ablösen von Gasblasen oder -ansammlungen mittels des erfindungsgemäßen Verfahrens oder der Freispülmethode ist in bestimmten erfindungsgemäßen Ausführungsformen an beliebigen Stellen des extrakorporalen Blutkreislaufs und von beliebigen Komponenten des extrakorporalen Blutkreislaufs möglich. Zu diesen Stellen oder Komponenten zählen der Blutfilter oder Dialysator. Zu diesen Stellen oder Komponenten zählen vor allem jene, durch welche - oder in welche hinein - eine erfindungsgemäß erzeugte Druckwelle läuft. Die vorliegende Erfindung ist somit nicht darauf beschränkt, Gasblasen oder -ansammlungen aus dem Gerinnselfänger abzulösen, auch wenn Teile der vorliegenden Beschreibung explizit auf einen Gerinnselfänger abgestellt sind, was aber nicht einschränkend zu verstehen ist. Auch andere Komponenten oder Bauteile des extrakorporalen Blutkreislaufs oder des Blutschlauchsets, wie beispielsweise der Blutfilter, können mittels des erfindungsgemäßen Verfahrens und/oder der erfindungsgemäßen Vorrichtungen von Gasblasen oder -ansammlungen befreit werden. Die vorliegende Erfindung erstreckt sich somit auch hierauf.

Einige oder alle erfindungsgemäßen Ausführungsformen können einen oder mehrere der oben oder im Folgenden genannten Vorteile aufweisen.

So führt das erfindungsgemäße Verfahren, im Gegensatz zum Vorsehen von Beschichtungen des Standes der Technik, welche die Entlüftbarkeit des Gerinnselfängers durch Modifikation der Benetzbarkeit des Werkstoffs des Gerinnselfängers verbessern sollen, vorteilhafterweise nicht zu einer generell erhöhten Passierbarkeit von Luftblasen durch den Gerinnselfänger; Letzteres wäre beim Vorwärtsbetrieb unerwünscht.

Das Verfahren kann während des Vorbereitens des Disposables, z. B. in Gestalt eines extrakorporalen Blutkreislaufs, oder der Behandlungsvorrichtung durchgeführt werden, ohne dass der Anwender eingreifen müsste.

Die Elimination von Restluft verhindert vorteilhaft eine mögliche unerwünschte Luftinfusion in der Behandlung (z. B. induziert durch Anlegen von Strömungsgeschwindigkeiten von Blutflüssen während der Behandlung, welche höher sind als Strömungsgeschwindigkeiten während der Vorbereitung der Vorrichtungen).

Die Elimination von Restluft im extrakorporalen System kann auch die Hämokompatibilität des Gesamtsystems vorteilhaft verbessern.

Die Steuerung der Entlüftung über die bei geschlossener Klemme auftretenden Drücke verhindert vorteilhaft ein Platzen des Schlauchsystems. Dies ist relevant, da Schlauchrollenpumpen bei Rückwärtsförderung ggf. erheblich höhere Okklusionsdrücke aufweisen als im Normalbetrieb.

Auch kann im Rahmen der Rückwärtsförderung die Funktion der arteriellen Druckmessung im Überdruckbereich getestet werden: Hierzu könnte z. B. vor Öffnung der venösen Klemme das Single-Needle-Ventil geschlossen werden. Im Gesamtsystem stellt sich dann ein positiver Restdruck ein; dieser kann mit arterieller und venöser Druckmessung bestimmt und verglichen werden.

Den Folgen eines erschwerten Durchtritts von Restluft durch die Maschen eines erfindungsgemäßen Gerinnselfängers, z. B. aufgrund vorhandenen Auftriebs, kann erfindungsgemäß ebenfalls vorteilhaft entgegen gewirkt werden.

Die vorliegende Erfindung wird im Folgenden anhand der beigefügten Zeichnungen, in welcher identische Bezugszeichen gleiche oder ähnliche Komponenten bezeichnen, exemplarisch erläutert. In den zum Teil stark vereinfachten Figuren gilt:
- Fig. 1: zeigt ein Beispiel eines Blutgerinnselfängers im Schnitt, aus welchem mittels des erfindungsgemäßen Verfahrens Gasanhaftungen gelöst werden können;
- Fig. 2: zeigt schematisch vereinfacht einen extrakorporalen Blutkreislauf zum Lösen von Gasanhaftungen von einem Blutgerinnselfänger.

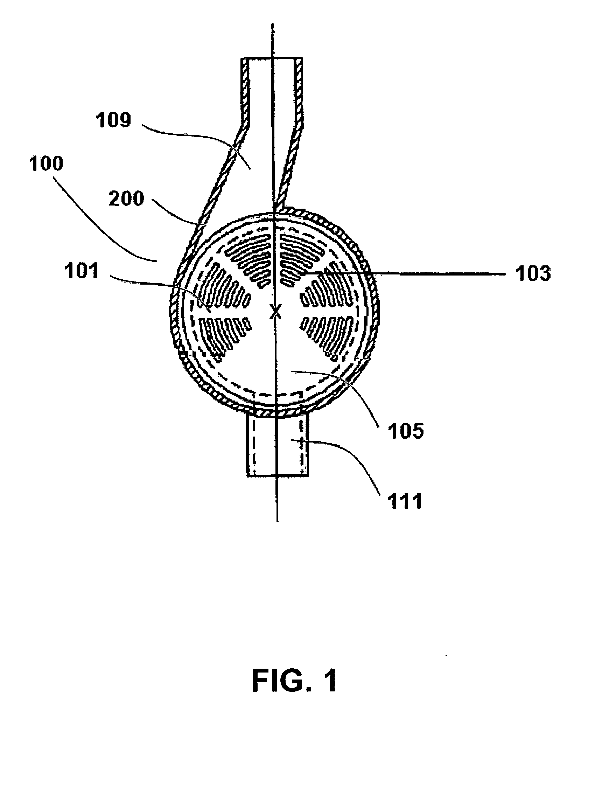

**Fig. 1** zeigt einen Schnitt durch einen in einem Gehäuse 200 angeordneten Blutgerinnselfänger (kurz auch Gerinnselfänger) 100. Der Gerinnselfänger 100 weist eine Siebfläche 101 auf, welche Durchtrittsöffnungen 103 und einen Abschnitt 105 ohne solche Durchtrittsöffnungen aufweist. Der Einströmbereich des Gerinnselfängers 100 ist mit dem Bezugszeichen 109, der Ausströmbereich mit 111 markiert. An den Durchtrittsöffnungen 103 können sich Luftbläschen halten. Diese können mittels des erfindungsgemäßen Verfahren von den Durchtrittsöffnungen gelöst werden.

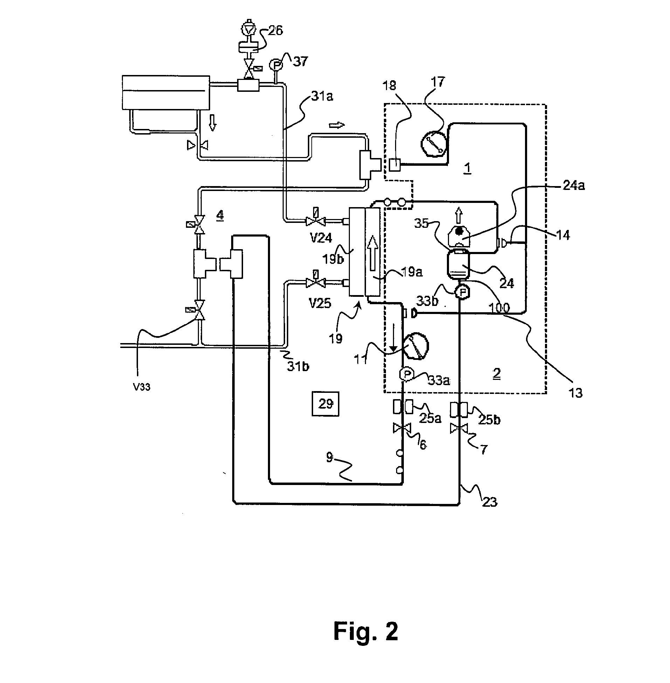

**Fig. 2** zeigt schematisch vereinfacht einen extrakorporalen Blutkreislauf 1, welcher wenigstens in Abschnitten hiervon Teil einer Blutkassette 2 ist. Zur Behandlung eines Patienten wird die Blutkassette 2 in eine medizinische Blutbehandlungsvorrichtung (kurz auch: Vorrichtung) 4 eingelegt.

Der extrakorporale Blutkreislauf 1 weist eine arterielle Patientenschlauchklemme 6 eines arteriellen Abschnitts 9, ferner eine venöse Patientenschlauchklemme 7 eines venösen Abschnitts 23 auf.

Eine Blutpumpe 11 ist im arteriellen Abschnitt 9 vorgesehen, eine Substituatpumpe 17 ist mit einer Substituatleitung verbunden. Die Substituatleitung kann mittels eines automatischen Substituatports 18 mit einer Substituatquelle verbunden werden. Mittels der Substituatpumpe 17 kann Substituat per Prädilution oder per Postdilution und die zugehörigen Zugabestellen 13 bzw. 14 in Leitungsabschnitte des Blutkreislaufs 1 eingebracht werden.

In den Blutkreislauf 1 ist ein Blutfilter 19 eingeschaltet. Dieser weist eine mit dem arteriellen Abschnitt 9 und mit dem venösen Abschnitt 23 verbundene Blutkammer 19a auf. Eine Dialysatkammer 19b ist mit einer zur Dialysatkammer 19b führenden Dialysatzulaufleitung 31a und einer von der Dialysatkammer 19b fortführenden Dialysatablaufleitung 31b verbunden. Der venöse Abschnitt 23 steht in Fluidkommunikation mit einer venösen Blutkammer 24 des extrakorporalen Blutkreislaufs 1.

Die venöse Blutkammer 24 steht an ihrer Ausströmseite mit dem Gerinnselfänger 100 in Fluidverbindung und/oder weist einen solchen auf.

Die venöse Blutkammer 24 steht an ihrer Einströmseite mit einem Single-Needle-Ventil 35 (SN-Ventil) in Fluidverbindung und/oder weist ein solches auf. Die venöse Blutkammer 24 steht an ihrer Einströmseite ferner mit einem Belüftungsventil 24a in Fluidverbindung und/oder weist ein solches auf.

Luftdetektoren 25a und 25b sind im arteriellen Abschnitt 9 bzw. im venösen Abschnitt 23 vorgesehen.

Eine erfindungsgemäße Steuerungseinrichtung 29 ist schematisch dargestellt. Sie steht in kontaktloser Signalverbindung (berührungslos) und/oder in Kontaktverbindung mit den zum Ausführen des erfindungsgemäßen Verfahren erforderlichen Komponenten wie Sensoren, Klemmen, Ventile, Blutpumpe usw.

Die Dialysatzulaufleitung 31a weist ein Ventil V24 auf, mittels welchem der Fluss innerhalb der Dialysatzulaufleitung 31a unterbunden werden kann. Die Dialysatablaufleitung 31b weist ein Ventil V25 auf, mittels welchem der Fluss innerhalb der Dialysatablaufleitung 31b unterbunden werden kann.

Die Dialysatzulaufleitung 31a ist ferner mittels eines weiteren, maschineninternen Ventils mit einer Druckluftquelle 26 verbunden.

Drucksensoren 33a und 33b messen den Druck im arteriellen Abschnitt 9 bzw. im venösen Abschnitt 23. Ein Drucksensor 37 misst den Druck in der Dialysatzulaufleitung 31a.

Der Dialysatkreislauf weist ein Spülventil V33 auf.

Zur Durchführung des erfindungsgemäßen Verfahrens werden in bestimmten Ausführungsformen in Anordnungen wie sie in Fig. 2 gezeigt sind der arterielle Abschnitt 9 und der venöse Abschnitt 23 miteinander kurzgeschlossen. Das Spülventil V33 kann geschlossen werden. Anschließend wird die Drehrichtung der Blutpumpe 11 umgekehrt. Die Blutpumpe 11 fördert Flüssigkeit gegen die zwischenzeitlich geschlossene venöse Patientenschlauchklemme 7 in der in Fig. 2 mit Pfeil angezeigten Richtung. Der hierbei aufgebaute Druck wird durch den in der Vorrichtung 4 vorhandenen Drucksensor begrenzt: Bei Erreichen eines festgelegten Maximaldrucks wird die venöse Patientenschlauchklemme 7 geöffnet. Der in der Compliance zwischen Blutpumpe 11 und venöser Patientenschlauchklemme 7 gefangene Druck baut sich schlagartig in die venöse Blutkammer 24 hinein ab. Er kann dort zu teils hohen und sehr hohen Spitzenflüssen führen, welche erheblich größer sind als jene Flüsse, welche bei Förderung mittels der Blutpumpe 11 im Normalbetrieb der Vorrichtung 4 in der ersten Förderrichtung auftreten. Aufgrund der Spitzenflüsse werden Luftblasen, die sich auf der Ausgangsseite (in Fig. 2 am unteren Ende des Gerinnselfängers) des Gerinnselfängers 100 befinden, über die Engstellen des Gerinnselfängers 100 hinweg vollständig oder zumindest teilweise mitgerissen.

In der hier vorgeschlagenen Ausführungsform des erfindungsgemäßen Verfahrens ist ein Einsatz der Substituatpumpe 17 nicht vorgesehen und/oder nicht erforderlich. Ventile der Zugabestellen 13 und 14 für Substituatflüssigkeit in Prädilution bzw. Postdilution (auch als Präventil und Postventil bezeichnet) sind geöffnet, um den Strömungswiderstand des Blutfilters 19, auch als Dialysator bezeichnet, zu umgehen. Das Single-Needle-Ventil 35 und das Belüftungsventil 24a sind geöffnet, um einen starken Unterdruckaufbau auf der Saugseite der Blutpumpe zu vermeiden. Um die Spitzenflüsse zu erhöhen, können das Single-Needle-Ventil 35 und/oder das Belüftungsventil 24a auch geschlossen sein, um den Druckgradienten über dem Gerinnselfänger 100 weiter zu erhöhen.

Der vorstehend beschriebene Entlüftungsvorgang kann - falls erforderlich - ein- oder mehrmals wiederholt werden.

Besonders wirksam ist das erfindungsgemäße Verfahren bei einer Anordnung des Gerinnselfängers derart, dass sich Luftblasen an einer Stelle ansammeln, an der sie bei Umkehr des Flusses durch die Strömung besonders ungehindert mitgerissen werden können.

In einer weiteren erfindungsgemäßen Ausführungsform erfolgt ein Freispülen des Gerinnselfängers 100 mittels einer Flüssigkeit, welche zu diesem Zweck der Hydraulik oder Hydraulikeinrichtung der Behandlungsvorrichtung 4 entnommen wird. Bei diesem Vorgehen können die Ventile V24 und/oder V25 geöffnet sein. Gleichzeitig können das Single-Needle-Ventil 35 und/oder das Belüftungsventil 24 geschlossen sein. Das Fluid wird auch in dieser Ausführungsform durch die rückwärts oder in der zweiten Strömungsrichtung fördernde Blutpumpe 11 gefördert. Diese baut ggf. auch den oben beschriebenen Überdruck auf.

Hiervon unterscheidet sich die im Folgenden beschriebene weitere erfindungsgemäße Ausführungsform. Bei dieser Ausführungsform werden - anders als in Fig. 2 gezeigt - der arterielle Abschnitt 9 und der venöse Abschnitt 23 vorteilhaft nicht miteinander kurzgeschlossen oder miteinander verbunden (obwohl dies auch bei dieser Ausführungsform durchaus möglich und ebenfalls von der vorliegenden Erfindung umfasst ist, beispielsweise bei rückwärts laufender Blutpumpe 11). Der Aufbau des erforderlichen Drucks des Fluids im Gerinnselfänger 100 wird in dieser Ausführungsform mittels eines Unterdrucks an der Membran des Blutfilters 19 erzeugt, welcher durch den Einsatz der Hydraulik der Behandlungsvorrichtung 4 erzielt wird.

### Verwendete Bezugszeichen:

- 1: extrakorporaler Blutkreislauf
- 2: Blutkassette
- 4: Vorrichtung oder medizinische Behandlungsvorrichtung
- 6: arterielle Patientenschlauchklemme
- 7: venöse Patientenschlauchklemme
- 9: arterieller Abschnitt
- 11: Blutpumpe
- 13: Zugabestelle für Substituatflüssigkeit (Prädilution)
- 14: Zugabestelle für Substituatflüssigkeit (Postdilution)
- 17: Substituatpumpe
- 18: automatischer Substituatport
- 19: Blutfilter oder Dialysator
- 19a: Blutkammer
- 19b: Dialysatkammer
- 23: venöser Abschnitt
- 24: venöse Blutkammer
- 24a: Belüftungsventil
- 25a: arterieller Luftdetektor
- 25b: venöser Luftdetektor
- 26: Druckluftquelle
- 29: Steuerungs- oder Regelungseinrichtung
- 31a: Dialysatzulaufleitung
- 31b: Dialysatablaufleitung
- 33a: Drucksensor
- 33b: Drucksensor
- 35: Single-Needle-Ventil
- 37: Drucksensor
- V24: Ventil
- V25: Ventil
- V33: Spülventil
- 100: Gerinnselfänger
- 101: Siebfläche
- 103: Durchtrittsöffnungen
- 105: Abschnitt ohne Durchtrittsöffnungen
- 109: Einströmbereich des Gerinnselfängers
- 111: Ausströmbereich
- 200: Gehäuse

## Patentansprüche

1. Verfahren zum Ablösen von Gasansammlungen von einem Gerinnselfänger (100) eines extrakorporalen Blutkreislaufs (1) vor Beginn einer Blutbehandlungssitzung , welcher während einer extrakorporalen Blutbehandlung in einer ersten Strömungsrichtung durchströmt oder angeströmt wird, mit den Schritten:
- Aufbauen eines Drucks in dem extrakorporalen Blutkreislauf (1) unter Einsatz einer Blutpumpe (11) ; und
- Schließen wenigstens eines Ventils zur Unterstützung des Aufbaus des Drucks,
**gekennzeichnet durch den weiteren Schritt:**
- Erzeugen eines Stroms eines Fluids **durch** den Gerinnselfänger (100) hindurch oder in diesen hinein in einer zweiten Strömungsrichtung, nachdem der aufgebaute Druck ein vorbestimmtes Druckniveau erreicht hat.

2. Verfahren nach Anspruch 1, mit dem Schritt:
- Kurzschließen eines arteriellen Abschnitts (9) des extrakorporalen Blutkreislaufs (1) mit einem venösen Abschnitt (23) des extrakorporalen Blutkreislaufs (1).

3. Verfahren nach Anspruch 1 oder 2, mit dem Schritt:
- Erzeugen des Stroms in der zweiten Richtung unter Einsatz einer Blutpumpe (11) oder unter Einsatz einer Hydraulikeinrichtung einer medizinischen Behandlungsvorrichtung (4).

4. Verfahren nach einem der vorangegangenen Ansprüche, mit dem Schritt:
- Überwachen der Höhe des aufgebauten Drucks oder des Druckgradienten mittels einer Einrichtung.

5. Verfahren nach einem der vorangegangenen Ansprüche, mit dem Schritt:
- Öffnen des Ventils einer Zugabestelle (13) für Substituatflüssigkeit zur Prädilution und/oder des Ventils einer Zugabestelle (14) für Substituatflüssigkeit zur Postdilution oder eines oder mehrerer weiterer Ventile zur Öffnung einer Kommunikationsverbindung mit einem Blutfilter (19).

6. Verfahren nach einem der vorangegangenen Ansprüche, mit dem Schritt:
- Öffnen eines Single-Needle-Ventils (35).

7. Verfahren nach einem der vorangegangenen Ansprüche, mit dem Schritt:
- Öffnen eines Entlüftungsventils (24a).

8. Verfahren nach einem der vorangegangenen Ansprüche, mit dem Schritt:
- Fördern von Fluid über eine Membran des Blutfilters (19) hinweg mittels oder unter Einsatz der Hydraulikeinrichtung der Behandlungsvorrichtung (4) als Fördereinrichtung.

9. Verfahren nach Anspruch 8, wobei mittels der Hydraulikeinrichtung der Behandlungsvorrichtung (4) ein Unterdruck über der Membran des Blutfilters (19) erzeugt wird.

10. Verfahren nach einem der vorangegangenen Ansprüche, mit dem Schritt:
- zwei- oder mehrmaliges Durchführen aller Schritte eines oder mehrerer der vorangegangenen Ansprüche.

11. Verfahren nach Anspruch 10, mit dem Schritt:
- Abbrechen des Verfahrens nach einer vorbestimmten Anzahl von durchgeführten Zyklen oder nachdem ein vorbestimmtes Volumen an Fluid mittels der Fördereinrichtung in der zweiten Strömungsrichtung gefördert wurde.

12. Steuerungseinrichtung (29), programmiert zum Durchführen eines Verfahrens nach einem der vorangegangenen Ansprüche.

13. Medizinische Behandlungsvorrichtung (4), welche wenigstens eine Steuerungseinrichtung (29) nach Anspruch 12 aufweist oder mit dieser in Signalübertragung steht oder zur Signalübertragung verbunden ist.

14. Medizinische Behandlungsvorrichtung (4) nach Anspruch 13, welche als Blutbehandlungsvorrichtung, insbesondere als Vorrichtung zur Apherese oder Dialyse, wiederum insbesondere zur Hämodialyse, Hämofiltration, Hämodiafiltration oder zur Akutdialyse, ausgestaltet ist.

## Claims

1. A method for removing gas accumulations before the beginning of a blood treatment session from a clot catcher (100) of an extracorporeal blood circuit (1), through which or against which fluid flows during an extracorporeal blood treatment in a first flow direction, with the steps:
- Building up pressure in the extracorporeal blood circuit (1) using a blood pump (11); and
- Closing at least one valve in order to support the pressure build-up,
characterized through the additional step:
- Generating a flow of a fluid through or into the clot catcher (100), in a second flow direction after the built-up pressure has reached a predefined pressure level.

2. The method according to claim 1, with the step:
- Bypassing an arterial section (9) of the extracorporeal blood circuit (1) with a venous section (23) of the extracorporeal blood circuit (1).

3. The method according to claim 1 or 2, with the step:
- Generating the flow in the second direction using a blood pump (11) or using a hydraulic device of a medical treatment apparatus (4).

4. The method according to one of the preceding claims, with the step:
- Monitoring the level of the built-up pressure or of the pressure gradient by means of a device.

5. The method according to one of the preceding claims, with the step:
- Opening the valve of an addition point (13) for substituate liquid for predilution and/or the valve of an addition point (14) for substituate liquid for postdilution or one or more further valves for opening a communication connection with a blood filter (19).

6. The method according to one of the preceding claims, with the step:
- Opening a single-needle valve (35).

7. The method according to one of the preceding claims, with the step:
- Opening a vent valve (24a).

8. The method according to one of the preceding claims, with the step:
- Conveying fluid across a membrane of the blood filter (19) by means of or utilizing the hydraulic device of the treatment apparatus (4) as conveying device.

9. The method according to claim 8, wherein an underpressure is produced across the membrane of the blood filter (19) by means of the hydraulic device of the treatment apparatus (4).

10. The method according to one of the preceding claims, with the step:
- Executing all steps of one or more of the preceding claims twice or several times.

11. The method according to claim 10, with the step:
- Aborting the method after a predefined number of performed cycles or after a predefined volume of fluid was conveyed in the second flow direction by means of the conveying device.

12. A control device (29) programmed for executing a method according to one of the preceding claims.

13. A medical treatment apparatus (4) which comprises at least one control device (29) according to claim 12 or which stands in signal transmission or is connected for signal transmission with it.

14. The medical treatment apparatus (4) according to claim 13 which is embodied as a blood treatment apparatus, in particular as an apparatus for apheresis or dialysis, again in particular for hemodialysis, hemofiltration, hemodiafiltration, or for acute dialysis.

## Revendications

1. Procédé pour détacher des accumulations de gaz d'un piège à caillots (100) d'une circulation sanguine extra-corporelle (1) avant le début d'une séance de traitement du sang, le piège à caillots (100) étant traversé ou exposé dans une première direction d'écoulement lors du traitement du sang, ledit procédé comprenant les étapes consistant à:
- générer une pression dans la circulation sanguine extra-corporelle (1) à l'aide d'une pompe à sang (11); et
- fermer au moins une vanne pour soutenir la montée en pression,
**caractérisé par** l'étape supplémentaire consistant à:
- provoquer un écoulement d'un fluide à travers ou dans le piège à caillots (100) dans une seconde direction d'écoulement après que la pression générée ait atteint un niveau de pression prédéterminé.

2. Procédé selon la revendication 1, comprenant l'étape consistant à:
- court-circuiter une section artérielle (9) de la circulation sanguine extra-corporelle (1) par une section veineuse (23) de la circulation sanguine extra-corporelle (1).

3. Procédé selon la revendication 1 ou 2, comprenant l'étape consistant à:
- provoquer l'écoulement dans la seconde direction d'écoulement à l'aide d'une pompe à sang (11) ou au moyen d'un dispositif hydraulique d'un appareil de traitement médical (4).

4. Procédé selon l'une quelconque des revendications précédentes, comprenant l'étape consistant à:
- surveiller le niveau de la pression générée ou le gradient de pression au moyen d'un dispositif de surveillance.

5. Procédé selon l'une quelconque des revendications précédentes, comprenant l'étape consistant à:
- ouvrir la vanne d'un point d'addition (13) de liquide de substitution pour prédilution et / ou la vanne d'un point d'addition (14) de liquide de substitution pour postdilution ou une ou plusieurs autres vannes pour l'ouverture d'une liaison de communication avec un filtre de sang (19).

6. Procédé selon l'une quelconque des revendications précédentes, comprenant l'étape consistant à:
- ouvrir une vanne Single-Needle (35).

7. Procédé selon l'une quelconque des revendications précédentes, comprenant l'étape consistant à:
- ouvrir une vanne d'aération (24a).

8. Procédé selon l'une quelconque des revendications précédentes, comprenant l'étape consistant à:
- transporter du fluide à travers une membrane du filtre à sang (19) au moyen de ou en utilisant le dispositif hydraulique de l'appareil de traitement (4) comme dispositif de transport.

9. Procédé selon la revendication 8, dans lequel une dépression est créée au niveau de la membrane du filtre de sang (19) au moyen du dispositif hydraulique de l'appareil de traitement (4).

10. Procédé selon l'une quelconque des revendications précédentes, comprenant l'étape consistant à:
- exécuter deux fois toutes les étapes de l'une ou de plusieurs des revendications précédentes.

11. Procédé selon la revendication 10, comprenant l'étape consistant à:
- interrompre le procédé après un nombre prédéfini de cycles effectués ou après qu'un volume prédéterminé de fluide ait été transmis dans la seconde direction d'écoulement au moyen du dispositif de transport.

12. Unité de commande (29) programmée pour exécuter un procédé selon l'une quelconque des revendications précédentes.

13. Appareil de traitement médical (4) comprenant au moins une unité de commande (29) selon la revendication 12, étant en transmission de signal avec celle-ci ou bien étant connecté avec celle-ci dans le but de transmettre des signaux.

14. Appareil de traitement médical (4) selon la revendication 13 configuré comme appareil de traitement du sang, en particulier comme appareil à aphérèse ou appareil de dialyse, en particulier d'hémodialyse, d'hémofiltration, d'hémodiafiltration ou de dialyse aigue.
